# EUROPÄISCHE PATENTANMELDUNG

(11) **EP 1 537 856 A1**
(43) Veröffentlichungstag der Anmeldung: **08.06.2005**
(21) Anmeldenummer: 04105498.2
(22) Anmeldetag: 03.11.2004
(51) Int. Cl.: A61K 7/48, A61K 7/075

(54) **Tensidhaltige Zubereitung mit Licochalcon A**

(30) Priorität: 03.12.2003 DE 10356869
(71) Anmelder: Beiersdorf AG, 20245 Hamburg (DE)
(72) Erfinder: Doerschner, Albrecht, 20146, Hamburg (DE); RUPPERT, Stephan, 20259, Hamurg (DE); ALBRECHT, Harald, 22083, Hamburg (DE); MUMMERT, Christopher, 29553, Bienenbüttel (DE); KOLBE, Ludger, 21255, Dohren (DE); KUETHER, JOERG, 25421, Pinneberg (DE); WILKEN, Maren, 22848 Norderstedt (DE); CERV, Svenja, 22761, Hamburg (DE)

(57) **Zusammenfassung**

Kosmetische Haar- und/oder Körperreinigungsmittel enthaltend Licochalcon A oder einen Extrakt aus Radix Glycrrhizae inflatae und ein oder mehrere Tenside neben gegebenenfalls weiteren kosmetischen und/oder dermatolgischen Wirk-, Hilfs- und Zusatzstoffen.

## Beschreibung

Die vorliegende Erfindung betrifft eine kosmetische und/oder dermatologische Zubereitung mit einem Gehalt an Licochalcon A und Tensiden, insbesondere zur Verbesserung des Zustandes gereizter Haut oder Kopfhaut, beispielsweise nach oder während der Anwendung einer solchen Zubereitung.
Der Wunsch nach einem sauberen und gepflegten Äußeren ist wohl so alt wie die Menschheit. Unreine Haut und ein ungepflegtes Haarkostüm bieten idealen Nährboden und Heimstatt für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Kosmetische und/oder dermatologische Reinigungspräparate werden in aller Regel in Form eines Schaums mit Wasser auf die zu reinigenden Körperpartien aufgetragen. Basis fast aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und ― je nach Wunsch ― auch für Schaumregulierung. Damit ist die Grundlage für die Schmutzentfernung lipidhaltiger Verschmutzungen gegeben.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Die meisten kosmetischen und dermatologischen Reinigungszubereitungen liegen in Form von mehr oder weniger viskosen Flüssigkeiten (Reinigungswässer, -gele und - milche) oder als Feststoff (z.B. Seife, Waschsynthete) vor. Sie werden in der Regel mit den Händen oder Fingern auf die Haut oder die Hautanhangsgebilde (z.B. Haare, Nägel) aufgetragen.

Zur Reinigung von keratinischen Fasern bzw. Haut werden i.A. Shampoos und Duschgele bzw. Gesichtsreiniger eingesetzt, die Tenside bzw. Tensidgemische und Pflegestoffe enthalten. An solche tensidhaltige Reinigungs- und/oder Rasiermittel (z.B. Shampoos, Duschgele, Rasierschäume etc.) werden eine Vielzahl von Anforderungen gestellt, die diese Zubereitungen gleichzeitig erfüllen sollen: Von diesen Zubereitungen wird erwartet, dass sie hautverträglich sind und ein angenehmes Hautgefühl nach der Anwendung auf er Haut zurücklassen. Diese Haut, insbesondere die Epidermis, ist als Barriereorgan des menschlichen Organismus in besonderem Maße äußeren Einwirkungen unterworfen. Derartige Einwirkungen können einen Reiz auf die Haut ausüben, die bei Menschen mit sensibler, empfindlicher oder verletzlicher Haut zu Rötung, Spannung, Brennen und / oder Juckreiz führen können. Zu solchen Einwirkungen zählen UV-Bestrahlung, mechanische Belastungen, Kontakt mit irritierenden Stoffen. Zu den Hautarealen, die diesen Einwirkungen weitgehend ungeschützt ausgesetzt sind und empfindlich reagieren gehört insbesondere die Kopfhaut. Neben der Sonnenbestrahlung der die Kopfhaut je nach Haartracht mehr oder weniger ungeschützt ausgesetzt ist, mechanischer Belastungen durch Kämmen, Bürsten, Schneiden inc. Rasieren, ist die Kopfhaut auch dem Kontakt mit irritierenden Stoffen ausgesetzt. Neben Chemikalien, die beim Haarfärben oder Dauerwellen eingesetzt werden, sind hier insbesondere die Tenside zu erwähnen, die beim häufig täglichen Haarwaschen zum Einsatz gelangen. Nach dem Haarwaschen wird das Haar und/oder die Kopfhaut häufig weiteren Behandlungen ausgesetzt die das Haar pflegen und die Kopfhaut stimulieren sollen, aber ebenfalls zu den beschriebenen unerwünschten Einflüssen führen.

Die eingesetzten Tenside und Tensidgemische setzen sich aus anionischen, amphoteren und/oder nichtionischen Tensiden zusammen. Bei den üblichen Einsatzkonzentrationen der Tenside bis ca. 25% und den für die Körper- und Haarreinigung üblichen Anwendungsbedingungen, weisen moderne Shampoozusammensetzung eine gute Hautverträglichkeit auf. Dennoch kann es bei empfindlicher Kopfhaut oder der vermehrten Anwendung (Sportler) zu Irritationen der Kopfhaut kommen. Der Einsatz von kleineren Einsatzkonzentrationen oder milderen Tensiden, stellt keine Lösung dieses Problems dar, da damit immer auch Leistungseinbussen bei dem Schaumvermögen und der Schaumcremigkeit verbunden sind.
Darüber hinaus sollen leicht aufschäumen und einen cremigen, feinblasigen Schaum bilden. Derartige Zubereitungen des Standes der Technik haben jedoch den Nachteil, dass sie diesen Anforderungen nur unzureichend gleichmäßig gerecht werden. Gut schäumende Produkte sind in der Regel wenig hautfreundlich und hinterlassen auf der Haut ein trockenes Gefühl. Besonders milde, hautfreundliche Produkte wiederum schäumen in der Regel schlecht.

Ein weiterer Nachteil am Stande der Technik besteht in dem Umstand, dass es, insbesondere in den Industrieländern, in der Bevölkerung zunehmend zu Unverträglichkeiten und allergischen Reaktionen gegenüber einer Vielzahl von Chemikalien und Inhaltsstoffen von Kosmtika bzw. Dermatika kommt. Von dieser Entwicklung sind in zunehmendem Maße auch tensidhaltige kosmetische und dermatologische Zubereitungen betroffen, da sie die schützende Lipidschicht auf der Haut teilweise ablösen und einerseits tief in die Haut einziehen, andererseits lange auf der Haut haftenbleiben können. Deshalb sollte die Konzentration von Tensiden in kosmetischen und/oder dermatologischen Zubereitungen möglichst niedrig gehalten werden. Ferner besteht ein Bedarf an tensidhaltigen Zubereitungen mit unterschiedlicher Tensidzusammensetzung, da Unverträglichkeiten und allergischen Reaktionen stoffspezifisch sind und den betroffenen Personen somit die Möglichkeit gegeben wird, kosmetische und/oder dermatolgische Zubereitungen mit einem auf ihr Unverträglichkeitsprofil abgestimmten Zusammensetzung zu benutzen.

Die Aufgabe der vorliegenden Erfindung war es die Mängel des Standes der Technik von tensidhaltigen kosmetischen und/oder dermatologsichen Zubereitungen, insbesondere von Haarshampoos, Duschgelen und Rasierschäumen, zu beseitigen oder zumindest deutlich zu reduzieren. Es sollten Zubereitungen entwickelt werden, die hautfreundlich sind und auf der Haut ein angenehmes Gefühl (weich, gepflegt, glatt, sauber) zurückzulassen. Gleichzeitig sollten diese Zubereitungen gut schäumen und einen cremigen, feinblasigen, dichten, stabilen Schaum bilden.

Licochalcone A ist Bestandteil des Auszugs aus Radix Glycrrhizae inflatae und zeichnet sich durch folgende Struktur aus:

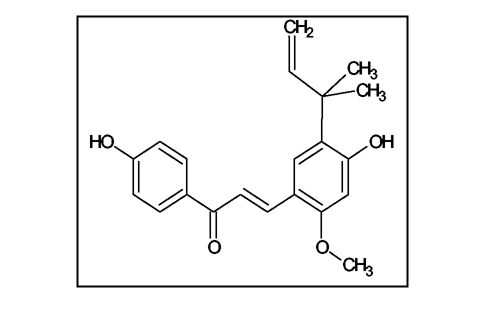

Die Pflanzenart Glycrrhizae inflatae gehört wie das in Europa offiizinelle Süßholz (Glycrrhiza glabra) der Gattung Glycrrhiza an, die zur Pflanzenfamilie der Fabaceae (Erbsengewächse) gehört.
Es hat sich nun überraschend und für den Fachmann nicht vorhersehbar herausgestellt, daß kosmetische Haar- und/oder Körperreinigungsmittel enthaltend Licochalcon A oder einen Extrakt aus Radix Glycrrhizae inflatae und ein oder mehrere Tenside, neben gegebenenfalls weiteren kosmetischen und/oder dermatolgischen Wirk-, Hilfs- und Zusatzstoffen den Nachteilen des Standes der Technik abhelfen. Durch solche Zubereitungen wird die Haut oder Kopfhaut, die mit dem Mittel bei der Anwendung in Kontakt kommt, besonders gepflegt indem der Zustand gereizter Haut oder Kopfhaut verbessert wird. Die Ursache gereizter Haut oder Kopfhaut kann UV-Bestrahlung, mechanische Beanspruchung und/oder Chemikalienkontakt sein. Insbesondere der Chemikalienkontakt und hierbei ist ganz besonders der häufige Kontakt mit Reinigungstensiden zu nennen ist ein häufig auftretender Stressfaktor für die Haut oder Kopfhaut, dessen Symptome, wie Rötungen, Brennen, Jucken, durch die erfindungsgemäßen Produkte deutlich gemindert oder beseitigt werden, ohne die Pflegeeigenschaften und die Qualität und Verteilbarkeit des Mittels in unguter Weise zu beeinflussen. Ein derart verbesserter Zustand ist besonders bei tensidgeschädigter Haut oder Kopfhaut zu beobachten. Der Extrakt aus Radix Glycrrhizae inflatae kann wässrig oder alkoholisch sein. Der alkoholische Extrakt ist aufgrund seiner Wirksamkeit besonders bevorzugt. Erhältlich sind solche Extrakte beispielsweise unter den Bezeichnungen "Polyol Soluble Licorice Extract P-U" oder "Aqua Licorice Extract P-U" der Fa. Maruzen Pharmaceuticals. Der erstgenannte Extrakt stellt einen alkoholischen Extrakt dar, der in Pulverform vorliegt. "Aqua Licorice Extract P-U" enthält noch zusätzliche ethoxilierte und/oder propoxilierte Rohstoffe. Dabei ist es von Vorteil, wenn diese ethoxilierten und/oder propoxilierten Rohstoffe, die vom Hersteller den Extrakten aus verschiedenen Gründen zugesetzt werden, in der Zubereitung nicht enthalten sind, weil deren mögliches Sensibilisierungspotential dann nicht in Erscheinung treten kann. Insbesondere Abwesenheit von PPG-6 (Nikkol PEN) ist von großem Vorteil. Ganz besonders vorteilhaft ist es, von einem alkoholischen Extrakt auszugehen, wie er unter der Bezeichnung Polyol Soluble Licorice Extract PU (INCI-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcon A. Es können somit kosmetische oder dermatologische Zubereitungen zur Körper- und Haarreinigung hergestellt werden, die eine hervorragende Milde aufweisen. Über diese Effekte hinaus konnte überraschend festgestellt werden, dass die erfindungsgemäßen Präparate vorteilhaft zur Behandlung und Prophylaxe bei postinflammatorischen Hautzuständen, ausgelöst durch Tensidschädigung der Haut, eingesetzt werden kann. Weiterhin sind diese Formulierungen für die Behandlung von Hautrötungen, insbesondere Rosacea, geeignet. Es ist bevorzugt, wenn der Gehalt an Licochalcon A oder Extrakt aus Radix Glycrrhizae inflatae mindestens 0,0001 Gew.%, besonders bevorzugt 0,0005 Gew.%, ganz besonders bevorzugt 0,001 Gew.%, ganz außergewöhnlich bevorzugt 0,01 Gew.%, ganz außergewöhnlich besonders bevorzugt 0,05 Gew.% und höchstens 10 Gew.%, besonders bevorzugt 5 Gew.%, ganz besonders bevorzugt bis 2 Gew.-%, ganz außergewöhnlich besonders bevorzugt 0,1 Gew.-% beträgt, jeweils bezogen auf das Gesamtgewicht der Zubereitung.
Weiterhin ist es bevorzugt, wenn als Tenside anionische, kationische, zwitterionische, amphotere Tenside, besonders bevorzugt anionische und zwittterionische Tensid und/oder die Kombination von ionischen Tensiden mit nichtionischen Tensiden eingesetzt werden. Besonders bevorzugt ist es, wenn die Gesamtmenge an Tensiden von 1 bis 50 Gewichts-%, besonders bevorzugt 5 bis 30 Gewichts-% bezogen auf das Gesamtgewicht der Zubereitung beträgt. Ganz besonders bevorzugt ist es, wenn das Verhältnis der Gesamtmenge an Sacchariden zur Gesamtmenge an Tensiden von 1:1000 bis 10:1 , bevorzugt von 1:300 bis 1:1 und ganz besonders bevorzugt von 1:100 bis 1:5 beträgt.
Bevorzugt ist es auch, wenn Natriumlaurethsulfat als Tensid allein oder in Kombination mit weiteren Tensiden eingesetzt wird, besonders bevorzugt die Kombination aus Natrium Laurylethersulfat und Cocamidopropyl Betain und/oder weiteren Tensiden, wie z. B. Alkylpolyglucosiden, Amphoacetaten, Sulfosuccinaten.
Bevorzugt ist es weiterhin, wenn ein solches Mittel in einem Schaumspender aufbewahrt und aus diesem heraus angewendet wird. Besonders bevorzugt ist es, wenn ein solches Mittel als Tränkung für ein unlösliches Substrat eingesetzt wird.
Bevorzugt sind auch solche Mittel, die sich als Haarreinigungsmittel eignen und zusätzlich ein polymeres quaternisiertes Ammoniumsalz der Hydroxyethylcellulose, welches mit einem trimethylammonium-substituierten Epoxid modifiziert ist (INCI: Polyquaternium-10) (Ucare Polymer JR 400 von Amerchol) und/oder ein depolymerisiertes Guar Gummi Derivat, welches quaternisiert ist (INCI: Guar Hydroxypropyl Trimonium Chlorid)(Jaguar Excel von Rhodia) in der Zubereitung vorliegt, besonders bevorzugt in Konzentrationen von 0,01 bis 5 Gew.%. Darüber hinaus ist eine Kombination aus einem solchen Mittel mit einem unlöslichen Substrat bevorzugt.
Die Erfindung umfasst auch die Verwendung von solchen Mitteln zur Behandlung, insbesondere zur Verbesserung des Zustandes vorgeschädigter Haut oder Kopfhaut, bevorzugt tensidgeschädigter Haut oder Kopfhaut, zur Reinigung und/oder Pflege der Haut und Hautanhangsgebilde, als Dusch-, Schaum- und/oder Wannenbad, als Haarshampoo, als Rasierschaum, als Gesichtsreinigungszubereitung zum Entfernen von dekorativer Kosmetik sowie ein Produkt umfassend ein solches Mittel enthalten in einem beschrifteten Packmittel, wobei die Beschriftung auf mindestens eine der genannten Verwendungen hinweist. Darüber hinaus ist ein nicht therapeutisches Verfahren zur Behandlung von tensidgeschädigter Haut oder Kopfhaut gekennzeichnet durch die topische Anwendung eines solchen Mittels.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.
Weitere vorteilhafte kationische Tenside im Sinne der vorliegenden Erfindung sind ferner
■ Alkylamine,
■ Alkylimidazole und
■ ethoxylierte Amine
und insbesondere deren Salze.

Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte nicht-ionische Tenside sind Alkohole und Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische Tenside eingesetzt werden wobei es bevorzugt ist, Natriumlaurethsulfat als Tensid allein oder in Kombination mit weiteren Tensiden einzusetzen. Besonderes bevorzugt sind diese Kombinationen, wenn die erfindungsgemäße Zubereitung als Shampoo, Duschgel, Schaum- oder Wannenbad sowie als Handwaschlotion eingesetzt wird.

Es ist erfindungsgemäß von Vorteil, wenn ein oder mehrere Tenside in einer Konzentration von 1 bis 50 Gewichts-%, bevorzugt in einer Konzentration 5 von 30 bis Gewichts-% und ganz besonders bevorzugt in einer Konzentration von 10 bis 20 Gewichts-%, jeweils bezogen auf das Gesamtgewicht der Zubereitung eingesetzt wird.

Anionische Tenside, bevorzugt Alkylsulphate, besonders bevorzugt ethoxylierte Alkylsulfate (Laurylethersulfate; Myristylethersulfate) werden erfindungsgemäß vorteilhaft in einer Konzentration von 1-20 Gewichts-% und bevorzugt in einer Konzentration von 5-12 Gewichts-%, bezogen auf das Gesamtgewicht der Zubereitung eingesetzt.
Diese Konzentrationsbereiche gelten auch als bevorzugt, wenn als anionische Tenside Seifen von Fettsäuren (Carbonsäuren und Derivate) eingesetzt werden, wie dies beispielsweise bei Rasierschäumen und -gelen der Fall ist.

Amphotere Tenside, bevorzugt Quaternäre Tenside, besonders bevorzugt Alkylamidopropylbetain, werden erfindungsgemäß vorteilhaft in einer Konzentration von 1-20 Gewichts-% und bevorzugt in einer Konzentration von 2-10 Gewichts-%, jeweils bezogen auf das Gesamtgewicht der Zubereitung eingesetzt.

Erfindungsgemäß bevorzugt werden diese Tenside in Kombination mit LES eingesetzt.

Erfindungsgemäß vorteilhaft können als amphotere Tenside auch Acyl/dialkylethylendiamine, insbesondere Dinatriumalkylamphodiacetat eingesetzt werden. Für diese amphoteren Tenside werden die bevorzugten Konzentrationsbereiche von 1-10 Gewichts-% und besonders bevorzugt von 1-5 Gewichts-%, jeweils bezogen auf das Gesamtgewicht der Zubereitung.

Ferner können Polysorbate als waschaktive Agentien erfindungsgemäß vorteilhaft in die Zubereitung eingearbeitet werden.
Im Sinne der Erfindung vorteilhafte Polysorbate sind dabei das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Ganz besonders vorteilhaft sind insbesondere
- Polyoxyethylen(20)sorbitanmonopalmitat (Tween 40, CAS-Nr. 9005-66-7)
- Polyoxyethylen(20)sorbitanmonostearat (Tween 60, CAS-Nr. 9005-67-8).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Formulierung einzeln oder als Mischung mehrer Polysorbate, eingesetzt.

Die erfindungsgemäße kosmetische und/oder dermatologische tensidhaltigen Zubereitungen ist auch dadurch gekennzeichnet, dass das Verhältnis der Gesamtmenge an Sacchariden zur Gesamtmenge an Tensiden von 1:1000 bis 10:1 , bevorzugt von 1:300 bis 1:1 und ganz besonders bevorzugt von 1:100 bis 1:5 beträgt.

Die erfindungsgemäße kosmetische und/oder dermatologische Zubereitung kann dabei neben einer oder mehrerer Wasserphasen zusätzlich eine oder mehrere Ölphasen enthalten und beispielsweise in Form von W/O- (Wasser in Öl-), W/S- (Wasser in Silikonöl-) O/W- (Öl in Wasser-) oder S/W-(Silikonöl in Wasser-) Emulsion vorliegen. Ferner können sie erfindungsgemäß vorteilhaft auch in sogenannten multiplen Emulsionen wie beispielsweise W/O/W-, O/W/O-, W/S/W- oder S/W/S-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion), eine sprühbare Emulsion oder eine Hydrodispersion sein. Des Weiteren können die Zubereitungen im Sinne der vorliegenden Erfindung auch nahezu wasserfrei sein (Wassergehalt unter 5 Gewichts-% bezogen auf das Gesamtgewicht der Zubereitung.

Die erfindungsgemäße Zubereitung kann als wässrige Phase neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Die Ölphase der erfindungsgemäßen Zubereitung, d.h. die lipophilen organischen Bestandteile, werden vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyl oleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (Cetiol OE) und/oder Dicaprylylcarbonat, beispielsweise das unter der Handelsbezeichnung Cetiol CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung Hallbrite BHB bei der Fa. CP Hall erhältliche), Hexadecylbenzoat und Butyloctylbenzoat und Gemische davon (Hallstar AB) und/oder Diethylhexylnaphthalat (Corapan®TQ von Haarmann & Reimer).

Auch beliebige Abmischungen solcher Öl- und Wachskomponenten sind vorteilhaft im Sinne der vorliegenden Erfindung einzusetzen.

Die Lipidphase kann die polaren Ölkomponenten erfindungsgemäß in einer Konzentration von bis zu 40 Gewichts-% bezogen auf das Gesamtgewicht der Lipidphase enthalten.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene und hydrierte Polyisobutene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 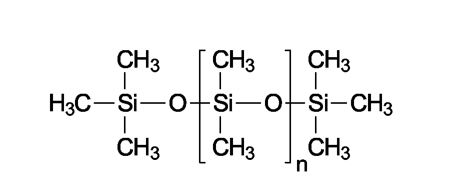 werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß vorteilhaft ist es, die lipophilen Bestandteile einer erfindungsgemäßen Zubereitung als rückfettende Substanzen zu verwenden. Als rückfettende Substanzen vorteilhaft einsetzbar sind beispielsweise zu wählen, beispielsweise Purcellinöl, Eucerit® und Neocerit®.

Ferner ist es erfindungsgemäß von Vorteil, wenn in den erfindungsgemäßen Zubereitungen Verdicker eingesetzt werden. Diese können beispielsweise vorteilhaft aus weiteren Verbindungen der Gruppe der Gummen gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Weiterhin erfindungsgemäß vorteilhaft ist die Verwendung von Polysacchariden und - derivaten.

Unter den Polysacchariden und -derivaten befinden sich z.B. Hyaluronsäure, Chitin und Chitosan, Chondroitinsulfate, Stärke und Stärkederivate als erfindungsgemäß vorteilhafte Verdicker.

Weiterhin erfindungsgemäß vorteilhaft ist die Verwendung von Cellulosederivaten.

Unter den Cellulosederivaten befinden sich z.B. Methylcellulose, Carboxymethylcellulose, Hydroxyethylcellulose, Hydroxypropylmethylcellulose als erfindungsgemäß vorteilhafte Verdicker.

Weiterhin erfindungsgemäß vorteilhaft ist die Verwendung von Schichtsilikaten.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form als Verdicker verwendet werden wie z.B. Stearylalkonium Hektorite.

Weiterhin können vorteilhaft auch Kieselsäuregele verwendet werden.

Weiterhin erfindungsgemäß vorteilhaft ist die Verwendung von Polyacrylaten.

Unter den Polyacrylaten befinden sich z.B. Carbopol Typen der Firma Noveon (Carbopol 980, 981, 1382, 5984, 2984, ETD 2001, ETD 2020, ETD 2050 oder Pemulen TR1 & TR2).

Weiterhin erfindungsgemäß vorteilhaft ist die Verwendung von Polymeren.

Unter den Polymeren befinden sich z.B. Polyacrylamide (Seppigel 305), Polyvinylalkohole, PVP, PVP / VA Copolymere, Polyglycole.

Es ist erfindungsgemäß von Vorteil ein oder mehrere Verdicker in einer Konzentration von 0,1 bis 5 Gewichts-%, bezogen auf das Gesamtgewicht der Zubereitung einzusetzen.

Ferner kann es erfindungsgemäß von Vorteil sein, wenn in den erfindungsgemäßen Zubereitungen Salze zur Verdickung der Zubereitung eingesetzt werden, so dass Zubereitungen mit Viskositäten von 2000 mPas bis 6000 mPas hergestellt werden können. Unter Salzen sind ein- oder mehrwerteige Alkalimetallverbindungen bzw. Erdalkalimetallverbindungen mit Halogenanionen zu verstehen. Besonders bevorzugt wird Natriumchlorid eingesetzt.

Es ist erfindungsgemäß von Vorteil ein oder mehrere Alkylisalze bzw. Erdalkylisalze in einer Konzentration von 0,1 bis 3 Gewichts-%, bezogen auf das Gesamtgewicht der Zubereitung einzusetzen.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2 -Chloracetamid, Benzalkoniumchlorid, Benzylalkohol, Salicylsäure und Salicylate.

Es ist dabei erfindungsgemäß besonders bevorzugt, wenn als Konservierungsstoffe lodopropylbutylcarbamate, Parabene (Methyl-, Ethyl-, Propyl- und/oder Butylparaben) und/oder Phenoxyethanol eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung enthalten.

Die erfindungsgemäße Zubereitung enthält vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Erfindungsgemäß vorteilhafte Konditionierer aus der Gruppe der Polyquaternium-Verbindungen können dabei beispielsweise aus den in der Tabelle aufgelisteten Verbindungen gewählt werden.

| **Bezeichnung nach INCI** | **CAS-Nummer** | **Polymertyp** | **Beispiel (Handelsname)** |
|---|---|---|---|
| Polyquaternium-2 | CAS 63451-27-4 | Urea, N, N'- bis[3-(dimethylamino)propyl]- , polymer mit 1, 1 '-oxybis(2- chloroethan) | Mirapol® A-15 |
| Polyquaternium-5 | CAS 26006-22-4 | Acrylamid, β-Methacryloxyethyltriethyl ammoniummethosulfat | |
| Polyquaternium-6 | CAS 26062-79-3 | N,N-Dimethyl-N-2-propenyl-2-propen-aminiumchlorid | Merquat® 100 |
| Polyquaternium-7 | CAS 26590-05-6 | N,N-Dimethyl-N-2-propenyl-2-propen-aminiumchlorid, 2-Propenamid | Merquat® S |
| Polyquaternium-10 | CAS 53568-66-4, 55353-19-0, 54351-50-7, 68610-92-4, 81859-24-7 | Quaternäres Ammoniumsalz der Hydroxyethylcellulose | Celquat® SC-230M |
| Polyquaternium-11 | CAS 53633-54-8 | Vinylpyrrolidon/dimethyla minoethyl-Methacrylat-Copolymer/Diethylsulfat-Reaktionsprodukt | Gafquat®755N |
| Polyquaternium-16 | CAS 29297-55-0 | Vinylpyrrolidon/vinylimid azolinummethochlorid-Copolymer | Luviquat® HM552 |
| Polyquaternium-17 | CAS 90624-75-2 | | Mirapol®AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinyloctadecylether | |
| Polyquaternium-21 | | Polysiloxan-polydimethyldimethylammoniumaceta t-Copolymer | Abil® B 9905 |
| Polyquaternium-22 | CAS 53694-17-0 | Dimethyldiallylammoniu mchlorid/Acrylsäure-Copolymer | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacryl amidopropyltrimethylam moniumchlorid-Copolymer | Gafquat®HS-100 |
| Polyquaternium-29 | CAS 92091-36-6, 148880-30-2 | Chitosan, das mit Propylenoxid umgesetzt u. mit Epichlorhydrin quaternisiert wurde | Lexquat® CH |
| Polyquaternium-31 | CAS 136505-02-7, 139767-67-7 | Polymeres, quaternäres Ammoniumsalz, das durch die Umsetzung von DMAPA-Acrylate/Acrylsäure/Acryl onitrogens-Copolymeren u. Diethylsulfat hergestellt wird | Hypan® QT 100 |
| Polyquaternium-32 | CAS 35429-19-7 | N,N,N-Trimethyl-2-{[82-methyl-1-oxo-2-propenyl)oxy]-ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |

Die erfindungsgemäß vorteilhaften kosmetischen Zubereitungen sind dadurch gekennzeichnet, dass das oder die Guar-Hydroxypropyltrimethylammoniumchloride eine Ladungsdichte von 0,4 bis 1,0 meq/g sowie ein Molekulargewicht von 100000 bis 1800000 aufweisen. Erfindungsgemäß bevorzugte Guar-Hydroxypropyltrimethylammoniumchloride stammen aus der Jaguar-Serie, erfindungsgemäß besonders bevorzugt ist das Produkt Jaguar Excel der Firma Rhodia.

Es ist erfindungsgemäß vorteilhaft ein oder mehrere mehrere Guar-Hydroxypropyltrimethylammoniumchloride in einer Konzentration von 0,01 bis 10 Gewichts-%, bevorzugt in einer Konzentration von 0,01 bis 2 Gewichts-% und ganz besonders bevorzugt in einer Konzentration 0,01 von bis 0,5 Gewichts-%, jeweils bezogen auf das Gesamtgewicht der Zubereitung einzusetzen.
Vorteilhaft im Sinne der vorliegenden Erfindung ist es, Polyquaternium-10 in einer Konzentration von 0.01 bis 2 Gewichts-%, bevorzugt in einer Konzentration von 0.05 bis 1.5 Gewichts-% und besonders bevorzugt von 0.1 bis 1.0 Gewichts-%, jeweils bezogen auf das Gesamtgewicht der Zubereitung, einzusetzen.
Desweiteren ist es vorteilhaft im Sinne der vorliegenden Erfindung Guarhydroxypropyltrimethylammoniumchlorid in Kombination mit Polyquaternium-10 in Konzentrationsverhältnissen von 1:10 bis 10:1 einzusetzen.

Ferner ist es im Sinne der vorliegenden Erfindung, der erfindungsgemäßen Zubereitung Perlglanzpigmente, Glimmer und/oder Effektpigmente zuzusetzen, um die Zubereitung optisch attraktiver zu gestalten.

Die erfindungsgemäßen Zubereitungen können erfindsungsgemäß vorteilhaft einen oder mehrere UV-Lichtschutzfilter enthalten.
Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate) und polymere UV-Filter wie das (3-(4-(2,2-bis-Ethoxycarbonylvinyl)-phenoxy) propenyl)- methylsiloxan/Dimethylsiloxan Copolymer, welches beispielsweise bei Hoffmann-La Roche unter der Handelsbezeichnung Parsol SLX erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ-505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazole Tetrasulfonate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.
- Hydroxybenzophenon-Derivate, wie z.B. 2-(4-Diethylamino-2-hydroxybenzoyl)-benzoic acid hexylester, welches beispielsweise von der Firma BASF unter dem Handelsnamen Uvinul® A Plus erhältlich ist.
- Benzoxazol-Derivate, wie z.B. das 2,4-bis-[5-1(dimethylpropyl)benzossazol-2-yl-(4-phenyl)-imino]-6-(2-ethylhexyl)-imino-1 ,3,5-triazine (CAS-Nr.: 288254-16-0), welches beispielsweise unter dem Handelsnamen UVASorb® K2A von der Firma 3V Sigma erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Diethylhexylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(tri methylsilyl )oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Die Liste der genannten UV-Filter, die im Sinne der vorliegenden Erfindung eingesetzt werden können, soll selbstverständlich nicht limitierend sein.

Die erfindungsgemäß vorteilhaften UV-Lichtschutzfilter werden bevorzugt in einer Konzentration von 0,1 bis 30 Gewichts-%, insbesondere in einer Konzentration von 0,5 bis 15 Gewichts-%, bezogen auf das Gesamtgewicht der Formulierung, eingesetzt.

Erfindungsgemäß vorteilhaft kann die erfindungsgemäße Zubereitung Glittersoffe und/oder andere Effektstoffe enthalten.

Vorteilhafte Ausführungsformen der erfindungsgemäßen kosmetischen Zubereitung sind auch dadurch gekennzeichnet, dass sie als weitere Bestandteile Trübungsmittel und/oder Perlglanzmittel enthalten. Als Trübungsmittel werden dabei erfindungsgemäß Substanzen und Substanzgemische verstanden, welche der Zubereitung ein trübes emulsionsartiges Aussehen verleihen.
Als Perlglanzmittel werden dabei erfindungsgemäß Substanzen und Substanzgemische verstanden, welche der Zubereitung ein opaleszierendes Aussehen verleihen.
Erfindungsgemäß vorteilhaft ist es auch Mischungen aus Trübungs- und Perlglanzmittel einzusetzen.
Erfindungsgemäß vorteilhafte Trübungsmittel/Perlglanzmittel bzw. Mischungen sind unter anderem:
■ PEG-3 Distearat (z.B. CUTINA TS der Firma Cognis),
■ eine Kombination aus Glycoldistearat, Glycerin, Laureth-4 und Cocamidopropylbetain (z.B. Euperlan PK 3000 und Euperlan PK 4000 der Firma Cognis),
■ eine Kombination aus Glycoldistearat, Cocosglucosiden, Glyceryloleat und Glycerylstearat (z.B. Lamesoft TM Benz der Firma Cognis).
■ Styrol/Acrylat Copolymere (z.B. Acusol OP 301 von Rohm & Haas)

Die erfindungsgemäße zubereitung kann vorteilhaft ein oder mehrere Antioxidantien enthalten. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 10 Gew.-%, besonders bevorzugt 0,025 - 2.0 Gew.-%, insbesondere 0.05 - 1.0 Gew.-%, bezogen auf das Gesamtgewicht der Zubereitung.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Antischuppenwirkstoffe (z.B. Selendisulfid, Zinkpyrithion, Pirocton, Olamin, Climbazol, Octopirox, Polydocanol und deren Kombinatinen), Antitranspirant-Salze (z.B. saure Aluminium- und/oder Aluminium/Zirkoniumsalze wie Aluminiumchlorhydrat und/oder Aluminium/Zirkoniumchlorhydrat), Pflanzen extrakte, Vitamine, Wirkstoffe, Peeling-Stoffe (Abrasiva, z.B. Polymerkügelchen oder -pulver aus Polyethylen, Polypropylen etc. anorganischen Oxiden, Silikaten usw.). Ferner können in der Zubereitung Haarglättungsmittel (z.B. Thioglycolate) enthalten sein.

Insbesondere ist es erfindungsgemäß von Vorteil der erfindungsgemäßen Zubereitung Vitamine, Pflanzenextrakte und UV-Lichtschutzfilter zuzusetzen. So ist beispielsweise der Zusatz von Calcium-Vitamin-Komplexen (z.B. aus γ-Oryzanol und Calciumsalzen wie Calciumpanthotenat, Calciumchlorid, Calciumacetat) erfindungsgemäß besonders vorteilhaft.

Außerdem können die erfindungsgemäßen Wirkstoffe (eine oder mehrere Verbindungen) auch sehr vorteilhaft gewählt werden aus der Gruppe der hydrophilen Wirkstoffe, insbesondere aus folgender Gruppe:
α-Hydroxysäuren wie Milchsäure oder Salicylsäure bzw. deren Salze wie z.B. Na-Lactat, Ca-Lactat, TEA-Lactat, Harnstoff, Allantoin, Serin, Sorbitol, Glycerin, Milchproteine, Panthenol, Chitosan, Polydocanol.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die in die erfindungsgemäßen Zubereitungen eingearbeiteten Wirkstoffe dienen unter anderem der Prophylaxe und/oder Behandlung von entzündlichen Hautzuständen und/oder zum Hautschutz bei empfindlich determinierter und trockener Haut (wie z. B. atopisches Ekzem, seborrhoisches Ekzem, polymorphe Lichtdermatose, Psoriasis, Vitiligo, Wundheilungsstörungen, Juckreiz, empfindlicher oder gereizter Haut, lichtbedingte Hautschäden und UV-induzierte Immunsuppression, Veränderungen der Desquamation, Veränderungen der normalen Fibroblasten- und Keratinozytenproliferation,Veränderungen der normalen Fibroblasten- und Keratinozytendifferenzierung defizitären sensitiven oder hypoaktiven Hautzustände oder defizitären sensitiven oder hypoaktiven Zustände von Hautanhangsgebilden und zur Verringerung der Hautdicke).

Die erfindungsgemäßen Zubereitungen stellen in jeglicher Hinsicht überaus befriedigende Präparate dar, welche nicht auf die in dieser Schrift erwähnte Rohstoffauswahl begrenzt sind.

Es ist erfindungsgemäß vorteilhaft, wenn die erfindungsgemäße kosmetische Zubereitung in einer Flasche oder Quetschflasche aufbewahrt und aus dieser heraus angewendet werden. Entsprechend sind auch Flaschen oder Quetschflaschen, welche eine erfindungsgemäße Zubereitung enthalten, erfindungsgemäß.

Erfindungsgemäß vorteilhaft kann die erfindungsgemäße Zubereitung als Tränkung auf ein Substrat aufgetragen sein. Die erfindungsgemäßen Substrate können glatt oder auch oberflächenstrukturiert sein. Erfindungsgemäß bevorzugt sind oberflächenstrukturierte Substrate.

Erfindungsgemäß ist auch die Kombination aus der erfindungsgemäßen kosmetischen und/oder dermatologischen Zubereitung und einem unlöslichen Substrat.

Bei den erfindungsgemäßen Substraten kann die Gewebebildung durch Kette und Schuss, durch Maschenbildung oder durch Verschlingung, und/oder kohäsive und/oder adhäsive Verbindung von Textilfasern erfolgen. Dabei ist es erfindungsgemäß bevorzugt, wenn es sich bei dem Substrat um ein Verbundstoff handelt.

Erfindungsgemäß bevorzugt werden Substrate in Form von Tüchern eingesetzt, welche aus Vlies bestehen, insbesondere aus wasserstrahlverfestigten und/oder wasserstrahlgeprägten Vlies. Die Substrate können vorteilhaft auch als Bausch, gelochtes Vlies oder Netz ausgeführt sein.

Derartige Substrate können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Es hat sich als vorteilhaft herausgestellt für das Tuch, wenn dieses ein Gewicht von 20 bis 120 g/m², vorzugsweise von 30 bis 80 g/m² besonders bevorzugt 40 bis 60 g/m² hat (gemessen bei 20 °C ± 2 °C und bei einer Feuchtigkeit der Raumluft von 65 % ± 5 % für 24 Stunden).

Die Dicke des Substrates beträgt vorzugsweise 0,2 mm bis 2 mm, insbesondere 0,4 mm bis 1,5 mm, ganz besonders bevorzugt 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer vorteilhaften Ausführungsform des Vlieses bestehen die Fasern aus einer Mischung aus 60 % bis 80 %Viskose mit 40% bis 20 % PET, insbesondere 70% Viskose und 30 % PET. Besonders vorteilhaft ist eine Mischung aus 70 %Viskose und 30 % PET.

Erfindungsgemäß vorteilhaft kann ein erfindungsgemäßes Vlies ein Gemisch aus drei verschiedenen Fasermaterialien aufweisen. In einem solchen Falle ist eine Mischung aus 40 % bis 80 %Viskose mit 50% bis 20 % PET und 1 bis 30% Baumwolle bevorzugt. Erfindungsgemäß besonders bevorzugt ist eine Mischung aus 40 %Viskose und 50 % PET und 10 % Baumwolle.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Ferner weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| im trockenen Zustand | Maschinenrichtung | 15 % bis 100 %, bevorzugt |
| | | 20 % und 50 % |
| | Querrichtung | 40 % bis 120 %, bevorzugt |
| | | 50 % und 85 % |
| im getränkten Zustand | Maschinenrichtung | 15 % bis 100 %, bevorzugt |
| | | 20 % und 40 % |
| | Querrichtung | 40 % bis 120 %, bevorzugt |
| | | 50 % und 85 % |

In einer erfindungsgemäß besonderen Darreichungsform derartiger erfindungsgemäßer Substrate, kann das Substrat nach der Imprägnierung mit der Zubereitung getrocknet werden um anschließend dem Anwender in Form eines trockenen Reinigungstuches dargereicht zu werden.

Die erfindungsgemäße Zubereitung kann vorteilhaft in einem Schaumspender aufbewahrt und aus diesem heraus angewendet wird. Bei dem Schaumspender kann es sich erfindungsemäß vorteilhaft um einen mechanischen Pumpspender (Pumpfoamer) oder um eine Aerosoldose handeln.

Die erfindungsgemäßen Zubereitungen können erfindungsgemäß vorteilhaft mit einem Treibgas aufgeschäumt werden. Dieses wird erfindungsgemäß in einer Menge von 0,5 bis 20 Gewichts-%, besonders vorteilhaft in einer Menge von 5 bis 15 und ganz besonders vorteilhaft in einer Menge von 8 bis 11 Gewichts-%, jeweils bezogen auf das Gesamtgewicht der Formulierung eingesetzt. Erfindungsgemäß besonders vorteilhafte Treibgase sind Propan, Isobutan und n-Butan sowie deren Mischungen.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Erfindungsgemäß ist die Verwendung der erfindungsgemäßen kosmetischen und/oder dermatologischen Zubereitung zur Reinigung und/oder Pflege der Haut und Hautanhangsgebilde.

Erfindungsgemäß ist die Verwendung der erfindungsgemäßen kosmetischen und/oder dermatologischen Zubereitung als Dusch-, Schaum- und/oder Wannenbad.

Erfindungsgemäß ist die Verwendung der erfindungsgemäßen kosmetischen und/oder dermatologischen Zubereitung als Haarshampoo.

Erfindungsgemäß ist die Verwendung der erfindungsgemäßen kosmetischen und/oder dermatologischen Zubereitung als Gesichtsreinigungszubereitung zum Entfernen von dekorativer Kosmetik.

Erfindungsgemäß ist Verwendung der erfindungsgemäßen kosmetischen und/oder dermatologischen Zubereitung zur Reinigung und Wäsche von Kleidungsstücken und Textilien ("Waschmittel").

Erfindungsgemäß ist Verwendung der erfindungsgemäßen kosmetischen und/oder dermatologischen Zubereitung zur Reinigung von Gegenständen des täglichen Lebens (z.B. Geschirr, Tisch- und Schrankflächen, Autos).

Erfindungsgemäß ist Verwendung der erfindungsgemäßen kosmetischen und/oder dermatologischen Zubereitung zur Reinigung und Pflege von Fellen und Haarkleidern von Säugetieren, insbesondere von Haus- und Nutztieren (z.B. Hunde, Katzen, Nagetiere, Pferde, Kühe, etc.).

Erfindungsgemäß ist Verwendung der erfindungsgemäßen kosmetischen und/oder dermatologischen Zubereitung zur Bekämpfung von Blatt- und Schildläusen, insbesondere wenn sich diese auf den Blättern von Zier- und Nutzpflanzen befinden.

Nicht zuletzt ist die Verwendung der kosmetischen oder dermatologischen Zubereitungen zur Prophylaxe und/oder Behandlung von entzündlichen Hautzuständen und/oder zum Hautschutz bei empfindlich determinierter und trockener Haut erfindungsgemäß.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Rezepturbeispiele:

Die hergestellten Rezepturen können klar oder mit Hilfe von Trübungsmittel und Perlglanz milchig, weiß hergestellt werden.

| **Haarshampoos** | | | | | | |
|---|---|---|---|---|---|---|
| | **1** | **2** | **3** | **4** | **5** | **6** |
| Natrium Laurylethersulfat | 9 | 8 | 9 | 9 | 9 | 9 |
| Cocamidopropyl Betain | 4 | 5 | 3 | 4 | 2 | 4 |
| Dinatrium PEG-5 Laurylcitrat Sulfosuccinat | 3 | 3 | 3 | 2 | 5 | - |
| Alkylpolyglukosid | - | - | - | - | - | 3 |
| Verdicker | - | - | 0.1 | 0.2 | 0.3 | 0.2 |
| Polyquaternium-10 | 0.3 | 0.1 | 0.1 | 0.3 | 0.1 | 0.1 |
| Guar Hydroxypropyl-Trimonium Chlorid | 0.1 | 0.1 | 0.1 | 0.2 | 0.2 | - |
| Licochalcone A | 0.025 | 0.025 | 0.05 | 0.02 | 0.1 | 0.025 |
| PEG-3 Distearat | 1.5 | 2 | 2 | 1.5 | 1.5 | - |
| Trübungsmittel | - | - | - | 0.1 | 0.5 | - |
| Iminodibersteinsäure | 0.1 | 0.2 | 0.1 | 0.5 | 0.5 | 0.5 |
| PEG-40 hydriertes Rizinusöl | 0.2 | 0.2 | 0.2 | 0.2 | 0.2 | 0.4 |
| Niacinamid | 0.01 | - | - | - | - | - |
| Jojobaöl | - | 0.01 | - | - | - | - |
| Vitamin E-acetat | - | - | 0.01 | - | - | - |
| Meeresmineralien | - | - | - | 0.01 | - | - |
| Panthenol | - | - | - | - | 0.01 | - |
| Natrium Salicylat | 0.4 | 0.4 | 0.4 | 0.4 | 0.4 | 0.4 |
| Calciumchlorid | | | | | | 0.05 |
| Oryzanol | | | | | | 0.05 |
| Natrium Benzoat | 0.4 | 0.4 | 0.4 | 0.4 | 0.4 | 0.4 |
| Natriumchlorid | 0.9 | 1.0 | 0.2 | - | - | 0.8 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

| **Hautreinigungsgele:** | | | | | |
|---|---|---|---|---|---|
| | **1** | **2** | **3** | **4** | **5** |
| Natrium Laurethsulfat | 13,2% | 11% | 9,5% | 11% | 9,5% |
| Cocoamidopropylbetain | 1,65% | 3,3% | 3,8% | 3,3% | 3,8% |
| PEG-7 Glyceryl Cocoate | --- | --- | --- | 2,0 % | 2,0 % |
| Laureth-2 | --- | --- | --- | 0,1 % | --- |
| PEG-90 Glyceryl Isosterate | --- | --- | --- | 0,3 % | --- |
| Natriumcocoylglutamat | 1,25% | 0,75% | 2,5% | 0,75% | 0,75% |
| PEG-40 hydriertes Rizinusöl | 0,50% | 0,50% | 0,5% | 0,50% | 0,50% |
| PEG-100 hydriertes Glycerylpalmitat | 0,50% | 0,50% | 0,5% | 0,50% | 0,50% |
| Licochalcone A | 0.025 % | 0.025 % | 0.05% | 0.02% | 0.1% |
| Polyquaternium-10 | 0,2% | --- | 0,2% | --- | --- |
| Natriumbenzoat | 0,45% | 0,45% | 0,45% | 0,45% | 0,45% |
| Natriumsalicylat | 0,20% | 0,20% | 0,2% | 0,20% | 0,20% |
| Citronensäure | 0,50% | 0,50% | 0,5% | 0,50% | 0,50% |
| Parfum | q.s. | q.s. | q.s. | q.s. | q.s. |
| Wasser | ad 100 | ad 100 | ad 100 | ad 100 | ad 100 |

| **LES-freie Hautreinigungsgele** | | | |
|---|---|---|---|
| | **1** | **2** | **3** |
| Natrium Myrethsulfat | 5% | 4% | 6% |
| Laurylglucosid | 2,5% | --- | --- |
| Decylglucosid | --- | 3% | --- |
| Natrium Cocoamphoacetat | 6,5% | 7% | 8% |
| PEG-200 hydriertes Glycerylpalmitat | 0,4% | 0,4% | 0,4% |
| PEG-40 hydriertes Rizinusöl | 1% | 1% | 1% |
| Diammonium Citrat | 0,12% | 0,12% | 0,12% |
| Polyquaternium-10 | 0,2% | --- | --- |
| Licochalcone A | 0.025 % | 0.025 % | 0.05% |
| Natriumbenzoat | 0,3% | 0,3% | 0,3% |
| Natriumsalicylat | 0,2% | 0,2% | 0,2% |
| Citronensäure | 1,2% | 1,2% | 1,2% |
| Parfum | q.s. | q.s. | q.s. |
| Wasser | ad 100 | ad 100 | ad 100 |

| **Gesichtsreinigungsgele** | | | | | |
|---|---|---|---|---|---|
| | **1** | **2** | **3** | **4** | **5** |
| Natrium Myrethsulfat | 2% | 4% | 3% | 5% | 2% |
| Decylglucosid | 2% | 2% | 4% | 1% | 4% |
| Cocoamidopropylbetain | --- | 2% | --- | 1% | 1% |
| Carbopol 1382 | 0,3% | 0,6% | 0,5% | 1% | -- |
| Acrylates Copolymer | 0,3% | 0,5% | 0,2% | 0,2% | 1% |
| Natriumhydroxid | 0,5% | 0,5% | 0,5% | 0,5% | 0,5% |
| Glycerin | 5% | 10% | 5% | 10% | --- |
| Na₃HEDTA | 0,5% | 0,5% | 0,5% | 0,5% | 0,5% |
| Licochalcone A | 0.025 % | 0.025 % | 0.025 % | 0.05% | 0.01 % |
| Phenoxyethanol | 0,5% | 0,5% | 0,5% | 0,5% | 0,5% |
| Parabene | 0,2% | 0,2% | 0,2% | 0,2% | 0,2% |
| Parfum | q.s. | q.s. | q.s. | q.s. | q.s. |
| Wasser | ad 100 | ad 100 | ad 100 | ad 100 | ad 100 |
| | | | | | |

| | **6** | **7** | **8** | **9** | **10** |
|---|---|---|---|---|---|
| Natrium Laurethsulfat | 2% | 2% | 7% | 7% | --- |
| Methyl Cocoyltaurat | 0,6% | 0,6% | 0,6% | 0,6% | 6% |
| Carbopol 980 | 1,2% | 1,2% | 1,2% | 0,5% | 0,6% |
| Natriumhydroxid | 0,5% | 0,5% | 0,5% | 0,5% | 0,5% |
| Glycerin | 2,0% | 2,0% | 2,0% | --- | 2,0% |
| Licochalcone A | 0.025% | 0.025% | 0.025% | 0.05% | 0.01% |
| Xanthan Gum | 0,25% | 0,1% | 0,25% | --- | --- |
| Phenoxyethanol | 0,5% | 0,5% | 0,5% | 0,5% | 0,5% |
| Parabene | 0,2% | 0,2% | 0,2% | 0,2% | 0,2% |
| Parfum | q.s. | q.s. | q.s. | q.s. | q.s. |
| Wasser | ad 100 | ad 100 | ad 100 | ad 100 | ad 100 |

| **Hautreinigungsemulsionen:** | | | | | |
|---|---|---|---|---|---|
| | **1** | **2** | **3** | **4** | **5** |
| Paraffinöl | 46% | 14% | 20% | 20% | 25% |
| Sojaöl | 24,3% | 36% | 20% | 20% | 25% |
| Natriumlaurylethersulfat | 7,35% | 12,3% | 11% | 11% | 11% |
| Natriumbenzoat | 0,3% | 0,3% | 0,3% | - | 0,3% |
| Natriumsalicylat | 0,2% | 0,2% | 0,2% | - | 0,2% |
| Acrylates/C10-C30 Alkyl Acrylate Crosspolymer | - | - | 1% | 1% | 0,8% |
| Licochalcone A | 0.025% | 0.025% | 0.05% | 0.02% | 0.1% |
| Natriumhydroxid | - | - | 0,2% | 0,2% | 0,2% |
| Phenoxyethanol | - | - | - | 0,5% | - |
| Parabene | - | - | - | 0,2% | - |
| Parfum | q.s. | q.s. | q.s. | q.s. | q.s. |
| Wasser | ad 100 | ad 100 | ad 100 | ad 100 | ad 100 |

| **Hautreinigungsöle** | | | |
|---|---|---|---|
| | **1** | **2** | **3** |
| Sojaöl | 40% | - | - |
| Rizinusöl | 14% | 14% | 9% |
| Sonnenblumenöl | - | 54% | - |
| Weizemkeimöl | - | - | 37% |
| Zetesol 100 | 41% | 30% | 51% |
| Licochalcone A | 0.025 % | 0.025 % | 0.05% |
| Poloxamer 101 | 2% | 2% | 2% |
| Parfüm, Antioxidantien, Konservierungsstoffe | q.s. | q.s. | q.s. |
| Wasser | ad 100 | ad 100 | ad 100 |
| | | | |

| | **4** | **5** | |
|---|---|---|---|
| TIPA-Laurethsulfat | 34% | 36% | |
| Rizinusöl | 30% | 30% | |
| Kokamid DEA | 10% | 10% | |
| PEG-40 Sorbitan Perisostearat | 10% | 15% | |
| Sojaöl | 9% | - | |
| Propylenglykol | 2% | 9% | |
| Licochalcone A | 0.025 % | 0.025 % | |
| Wasser | 1% | - | |
| Parfüm, Antioxidantien, Konservierungsstoffe | q.s. | q.s. | |

| **Pumpfoamer** | | | | |
|---|---|---|---|---|
| | **1** | **2** | **3** | **4** |
| Natrium Cocoylglutamat | 2,5 | - | - | - |
| Natrium Laurylethersulfat | - | 3,5 | - | - |
| Natrium Lauroylsarkosinat | - | - | 5 | - |
| Natrium Myristylethersulfat | - | - | - | 4,5 |
| Decylglucosid | 3 | 4 | - | - |
| Laurylglucosid | - | - | 3 | 3 |
| Polyquaternium-10 | 0,1 | - | - | 0,1 |
| Guar Hydroxypropyltrimoniumchlorid | - | 0,15 | - | - |
| Polyquaternium-22 | - | - | 0,2 | - |
| PEG-200 hydriertes Glycerylpalmitat | 0,5 | - | - | - |
| PEG-40 hydriertes Rizinusöl | 0,1 | 0,1 | 0,1 | 0,1 |
| PEG-100 hydriertes Glycerylpalmitat | - | 0,5 | - | 0,5 |
| Licochalcone A | 0.025 | 0.025 | 0.05 | 0.02 |
| Natriumbenzoat | 0,5 | 0,5 | - | 0,5 |
| Natriumsalicylat | - | 0,2 | - | 0,2 |
| Phenoxyethanol | - | - | 0,16 | - |
| Jojobaöl (Buxus Chinensis) | 0,1 | - | - | - |
| Citronensäure | q.s. | q.s. | q.s. | q.s. |
| Parfüm | q.s. | q.s. | q.s. | q.s. |
| Wasser | ad 100 | ad 100 | ad 100 | ad 100 |

| **Nachschäumende Hautreinigungsformulierungen:** | | | | | |
|---|---|---|---|---|---|
| | **1** | **2** | **3** | **4** | **5** |
| Hydroxypropyl Stärke Phosphat Ester (Structure XL) | 1% | 2% | 5% | 1% | 7% |
| Natrium Laurethsulfat | 18,0 | 12,0 | 8,0 | 6,0 | 10,0 |
| Sorbit | 2,0 | 0 | 3,0 | 3,0 | 5,0 |
| Laureth-4 | 0 | 0 | 7,0 | 7 | 0 |
| Lanolinalkohol-PEG15 | 7,0 | 0 | 0 | 0 | 4,0 |
| Isopropylpalmitat | 0 | 0 | 3,0 | 3,0 | 5,0 |
| Isopropylmyristat | 4,0 | 2,0 | 0 | 0 | 0 |
| Glycerin | 0 | 5,0 | 2,0 | 1,0 | 0 |
| Xanthan Gum | 0,5 | 0,5 | 0 | 0 | 0 |
| Phenoxyethanol | 0,2 | 0 | 0,2 | 0 | 0 |
| Parabene | 0,7 | 0 | 0,5 | 0 | 0 |
| Natriumhydroxid | 0,5 | 0 | 0,1 | 0,1 | 0 |
| Na₃HEDTA | 0,5 | 0 | 0,5 | 0 | 0 |
| Licochalcone A | 0.1 | 0.025 | 0.05 | 0.02 | 0.1 |
| Natriumbenzoat | 0 | 0 | 0,0 | 0,5 | 0,5 |
| Zitronensäure | 0 | 0,2 | 0,5 | 0,5 | 0 |
| Polyquaternium-10 | 0 | 0 | 0,2 | 0,2 | 0,2 |
| Natriumsalicylat | 0 | 0,5 | 0 | 0 | 0 |
| Farbstoff | 0 | 0 | 0 | 0,5 | 0,3 |
| Gas / Aufschäummittel | 10,0 | 5,0 | 12,0 | 9,0 | 15 |
| Parfum | q.s. | q.s. | q.s. | q.s. | q.s. |
| Wasser | ad 100 | ad 100 | ad 100 | ad 100 | ad 100 |

## Patentansprüche

1. Kosmetische Haar- und/oder Körperreinigungsmittel enthaltend Licochalcon A oder einen Extrakt aus Radix Glycrrhizae inflatae und ein oder mehrere Tenside, neben gegebenenfalls weiteren kosmetischen und/oder dermatolgischen Wirk-, Hilfs- und Zusatzstoffen.

2. Mittel nach einem der vorangehenden Ansprüche **dadurch gekennzeichnet, daß** der Gehalt an Licochalcon A oder der Extrakt aus Radix Glycrrhizae inflatae 0,0001 bis 5 Gew.%, besonders bevorzugt 0,005 bis 2 Gew.%, ganz besonders bevorzugt 0,01 bis 0,1 Gew.-% bezogen auf das Gesamtgewicht der Zubereitung, beträgt.

3. Mittel nach einem der vorangehenden Ansprüche **dadurch gekennzeichnet, dass** als Tenside anionische, kationische, zwitterionische, amphotere Tenside, besonders bevorzugt anionische und zwittterionische Tensid und/oder die Kombination von ionischen Tensiden mit nichtionischen Tensiden eingesetzt werden.

4. Mittel nach einem der vorangehenden Ansprüche, **dadurch gekennzeichnet, dass** die Gesamtmenge an Tensiden von 1 bis 50 Gewichts-%, besonders bevorzugt 5 bis 30 Gewichts-% bezogen auf das Gesamtgewicht der Zubereitung beträgt.

5. Mittel nach einem der vorangehenden Ansprüche, **dadurch gekennzeichnet, dass** das Verhältnis der Gesamtmenge an Sacchariden zur Gesamtmenge an Tensiden von 1:1000 bis 10:1 , bevorzugt von 1:300 bis 1:1 und ganz besonders bevorzugt von 1:100 bis 1:5 beträgt.

6. Mittel nach einem der vorangehenden Ansprüche, **dadurch gekennzeichnet, dass** Natriumlaurethsulfat als Tensid allein oder in Kombination mit weiteren Tensiden eingesetzt wird, besonders bevorzugt die Kombination aus Natrium Laurylethersulfat und Cocamidopropyl Betain und/oder weiteren Tensiden, wie z. B. Alkylpolyglucosiden, Amphoacetaten, Sulfosuccinaten.

7. Mittel nach einem der vorangehenden Ansprüche, **dadurch gekennzeichnet, dass** sie in einem Schaumspender aufbewahrt und aus diesem heraus angewendet wird.

8. Mittel nach einem der vorangehenden Ansprüche, **dadurch gekennzeichnet, dass** sie als Tränkung für ein unlösliches Substrat eingesetzt wird.

9. Haarreinigungsmittel nach einem der vorangehenden Ansprüche **dadurch gekennzeichnet, daß** zusätzlich ein polymeres quaternisiertes Ammoniumsalz der Hydroxyethylcellulose, welches mit einem trimethylammonium-substituierten Epoxid modifiziert ist und/oder ein depolymerisiertes Guar Gummi Derivat, welches quaternisiert ist in der Zubereitung vorliegt, besonders bevorzugt in Konzentrationen von 0,01 bis 5 Gew.%.

10. Kombination aus einem Mittel nach einem der vorangehenden Ansprüche und einem unlöslichen Substrat.

11. Verwendung von Mitteln nach einem der vorangehenden Ansprüche zur Behandlung, insbesondere zur Verbesserung des Zustandes vorgeschädigter Haut oder Kopfhaut, bevorzugt tensidgeschädigter Haut oder Kopfhaut.

12. Verwendung von Mitteln nach einem der vorangehenden Ansprüche zur Reinigung und/oder Pflege der Haut und Hautanhangsgebilde, als Dusch-, Schaum- und/oder Wannenbad, als Haarshampoo, als Rasierschaum, als Gesichtsreinigungszubereitung zum Entfernen von dekorativer Kosmetik.

13. Produkt umfassend ein Mittel nach einem der vorangehenden Ansprüche enthalten in einem beschrifteten Packmittel, wobei die Beschriftung auf die Verwendung nach Anspruch 11 und/oder 12 hinweist.

14. Nicht therapeutisches Verfahren zur Behandlung von tensidgeschädigter Haut oder Kopfhaut **dadurch gekennzeichnet, daß** ein Mittel nach einem der vorangehenden Ansprüche topisch angewandt wird.
